# EUROPEAN PATENT SPECIFICATION

(11) **EP 1 912 731 B1**
(45) Date of publication and mention of the grant of the patent: **29.11.2017**
(21) Application number: 06765169.5
(22) Date of filing: 31.07.2006
(51) Int. Cl.: B01J 19/00, G01N 33/552, C12Q 1/68

(54) **MANUFACTURE OF ARRAY CHIPS**
HERSTELLUNG VON ARRAY-CHIPS
FABRICATION DE PUCES DE RÉSEAU

(30) Priority: 29.07.2005 GB 0515695
(43) Date of publication of application: 23.04.2008
(73) Proprietor: Randox Laboratories Ltd., Crumlin, Co. Antrim Northern Ireland, BT29 4QY (GB)
(72) Inventor: BOTA, Sorin, Randox Laboratories Ltd., Northern Ireland BT29 4QY (GB); ARIKAINEN, Ekaterina, Randox Laboratories Ltd., Northern Ireland BT29 4QY (GB); MCCONNELL, Robert Ivan, Randox Laboratories Ltd., Northern Ireland BT29 4QY (GB); FITZGERALD, Stephen Peter, Randox Laboratories Ltd, Northern Ireland BT29 4QY (GB)
(74) Representative: Gill Jennings & Every LLP
(86) International application number: PCT/GB2006/002856
(87) International publication number: WO 2007/012885

(56) References cited:
- EP-A- 1 273 349
- WO-A2-02/081743
- US-A- 5 682 242
- US-A1- 2003 082 587
- US-B1- 6 399 463
- Rudolf F. Graf: "Scribe and Break, Scribing" In: "Modern Dictionary of Electronics, 7th revised edition", 1 January 1999 (1999-01-01), Newness, Elsevier, XP055109504, ISBN: 978-0-75-069866-5 page 671,

## Description

### Field of the Invention

This invention relates to methods of improving quality control in the production of analytical arrays.

### Background to the Invention

Micro-array technology allows a large number of experiments to be performed simultaneously on a single substrate, commonly known as a biochip when used for biological analytes. The need for ever-faster and higher-throughput genomic and proteomic analysis has driven the development of array chips, sometimes referred to as biochips, protein chips or DNA chips, which are now sufficiently accurate and precise to be used in clinical diagnosis. For example, the Randox Evidence™ biochip allows multiple clinical diagnostic tests to be conducted simultaneously on a single patient sample.

Micro-arrays are, essentially, a series of ligands immobilised onto discrete areas of a substrate which are used to capture a specific analyte present in a biological sample. The quality of any micro-array is therefore dependent upon the quality (e.g. stability, purity) of the reagents used, the quality and uniformity of the substrate surface and the performance of the equipment (the "arrayer" or "spotter") used to deposit the ligands onto the substrate. Control over the atmospheric and incubation conditions in the array chip production process is also critically important. Although the quality and stability of the reagents prepared for deposition ("spotting") can be tested and ensured independently while in solution, ensuring perfect performance of the surface and the spotter is much more difficult in practice, if not impossible.

Regarding control over the substrate quality, surface tension deviations in some areas of substrate can be very difficult to identify by non-invasive methods. With regard to the arrayer performance, there is no way of ensuring absolutely that the performance is fault free over long periods, particularly under conditions of high production throughput. For example, deviations of the spot shape due to misdirected depositions from a piezo-tip bio-arrayer, ("spraying") is one of the most common types of defects occurring in array chip manufacture.

Each individual chip is normally produced from a larger piece of substrate material, known as a wafer. It is essential that, at the point of extraction from the wafer, each chip is subjected to rigorous quality control, to ensure that only those chips with the required parameters go into the next stage of manufacture.

It is essential for the mass production of array chips that quality control is total in its nature, whereby every single deposition spot is tested for compliance with the pre-defined quality/acceptability/array consistency criteria, including size, shape, spot position and degree of donut shape.

Assay performance in a array chip format is intimately linked to the amount, activity and stability of the ligands deposited . Ensuring the reproducibility of the deposition buffer therefore ensures reproducibility of the assay results.

Spotting buffers used to deliver ligands onto the chip surface are typically water-based mixtures of the following main components: bases, acids, various sodium salts, sugars, polysaccharides, surfactants, pH stabilisers, amino acids and proteins. The roles of various additives to the spotting buffers are: promotion of ligand immobilisation on the surface, stabilisation of proteins in the spotting solution and at the substrate surface, adjustment of surface tension, or drying rate of the microspot and reduction of patterns of ring or "donut" formation (the fundamentals of the ring-formation in a drying droplet are explained by Deegan R. D., et al in Nature, 389, 827-829, 1997; minimisation of protein adsorption on glass capillaries of the spotter is explained in Delehanty J. B. and Ligler F. S., Biotechniques, 34, 380-385, 2003).

Spotting buffers made up from the usual components, as listed above, are colourless and the buffer solution deposits are practically invisible shortly after spotting. Introduction of dyes into spotting buffers offers an opportunity to visualise bioarrays.

The use of fluorescent dyes and fluorescent nucleic acid stains in DNA arrays as a means of inducing contrast between the spots and the substrate are known, see for example US Patent 6,558,623; and J. E. Forman, et al., Methods in Enzymology, 2003, 361, pp. 530-548; J. R. Shearstone, et al., BioTechniques, 32, 1051-57, 2002. However, there are fundamental problems associated with fluorescence, including excitation intensity fluctuations, absorption by the sample, auto-fluorescence, fluorescence of the substrate and quenching.

Protein staining methods for adsorbed/immobilised protein quantification are disclosed in, for example, Lewis W. S. and Schuster S. M., J. Biochem. Methods, 21, 129-144, 1990. Dyes such as Coomassie Blue G-250 or R-250 are known to bind to proteins at acidic pH, where an electrostatic attraction is created between the dye and the protein.

The use of reference marks on the substrate onto which the ligand is deposited has been used to increase the accuracy with which discrete regions of a substrate or an array can be accessed. Examples of this are given in US 6,362,004, US 6,673,315 and US 6,356,343. However, precise measurement of spot positions can be difficult across large substrates/sheets. Normally, this depends on the ability to determine accurately the positions of the sheet edges and subsequently the positions of spots on the sheet with reference to these edges. There remains the need for a method to detect accurately whether or not a ligand has been deposited correctly onto a substrate.

Each spot in an array is potentially crucial and there is therefore an ongoing need to improve the quality control over array chip production.

### Summary of the Invention

This invention is based on the realisation that scribing at least one scribe line into the surface of a wafer substrate, that is the reverse of the surface onto which a ligand will be deposited, allows for an improved array chip production process. The inventors have further realised that the detection of the deposition of a ligand onto a substrate can be improved by adding to the deposition solution a visible dye that does not interact chemically with the ligand. This allows each deposit to be monitored simply, using an optical device such as a camera. This improves the quality control that is possible when monitoring the production of an array.

According to a first aspect of the invention, a method of manufacturing an array chip, comprises the steps of:
i) depositing at least one ligand onto the active surface of a wafer substrate, having at least one scribe line, the active surface being the reverse of the surface comprising the at least one scribe line;
ii) detecting the position of the deposited ligand in relation to the at least one scribe line;
iii) determining whether the ligand has been deposited correctly in relation to the at least one scribe line; and
iv) breaking the wafer substrate along the at least one scribe line, to produce at least one array chip.

### Description of the Drawings

The invention is described with reference to the following drawings, wherein:
Figure 1 illustrates a wafer substrate on which are spotted a number of 5 x 5 arrays on the upper surface, the grid pattern illustrates the scribe lines on the
   underneath of the substrate which sub-divides the substrate into potential individual biochips (each 5 x 5 array therefore appears within its own biochip area);
Figure 2 shows the use of the scribe lines as a positional reference allowing the spot positions to be determined with respect to a specific corner of cach biochip (also note that the small rectangular scribe mark in the bottom left hand corner of each biochip provides a reference for additional visual check if required in (other) QC processes);
Figure 3 illustrates the main elements of a system for monitoring deposition of ligand onto a substrate using scribe lines, wherein the substrate is mounted on an XY stage which is moved under PC control, a narrow beam (point) laser source/sensor device provides a laser spot focused on the scribed side of the substrate (the position and direction of the laser/sensor are fixed) and the opposite i.e dye spotted side of the substrate is viewed by a fixed solid-state pixel array camera system that is focused on the region of the substrate which surrounds the laser spot position;
Figure 4 illustrates one pattern of movement of the substrate with respect to the fixed laser sensor and fixed camera wherein the progressive zigzag pattern across the chip provides 10 intersections of the vertical scribe lines and two intersections of the horizontal scribe lines;

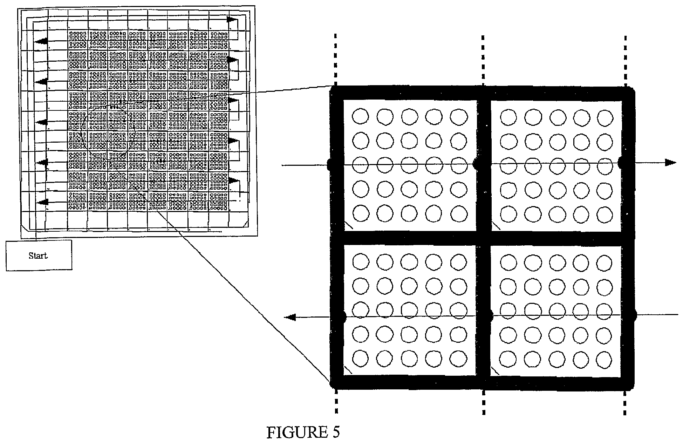

Figure 5 illustrates a magnified view of a small region on the substrate showing the sight offset errors which can occur one or other side of the scribe mark depending on the direction of scan;
Figure 6 illustrates a pattern of movement in which every scribe line is crossed at least 4 times during one complete scan of each on the biochip areas on the substrate;
Figure7 illustrates the use of a second laser(s) in the set-up procedure to determine the separation from the camera and adjust the distance between them, wherein the second laser illuminates the (upper) surface of the substrate at an accurate angle to the camera axis and movement of the substrate towards or away from the camera therefore is observed by a shift in the spot position observed by the camera; Observation of the spot image therefore allows adjustments to be made to the distance between the substrate and camera;
Figure 8 illustrates a laser scribe pattern;
Figure 9 illustrates all positions detected as crossing points by a scribing detection laser; and
Figure 10 illustrates determining the angle by which the wafer substrate has offset with reference to the scanning pattern.

### Detailed Description of the Invention

The present invention provides methods that improve the production of array chips.

The invention is based on the realisation that preparing array chips from a wafer substrate that has been scribed on the reverse of the active surface is advantageous.

As used herein, the term "array chip" refers to a support surface comprising a plurality of ligands deposited thereon. Preferably the support surface is solid. Suitable support materials are well known in the art, including surfaces with reflective, semitransparent, transparent, absorbing properties, or with low fluorescent background. A specific surface porosity or roughness is not required. The substrate should not absorb all visible light. Substrates can be activated for immobilisation of a ligand, for example by silanation, which is known in the art. Suitable substrates will be apparent to the skilled person, for example silicon, glass or ceramic materials may be used.

In the most preferred embodiment, the array chip is ceramic and a preferred ceramic material is alumina (also known as aluminium hydroxide, Al₂O₃).

The array chips according to the invention are produced from a larger support material, referred to herein as the wafer substrate. A plurality of array chips can be produced from a single wafer substrate, as is known in the art.

The wafer substrate, and therefore the array chips that derive from the wafer substrate, has a surface onto which the ligands will be attached. This is referred to herein as the "active" surface. The present invention requires scribing at least one scribe line onto the reverse of this surface. The scribing is carried out before the ligands have been attached to the active surface. It is preferred that scribing occurs first, preferably followed by cleaning to remove debris, activation of the active surface (which has not been scribed) eg. by silanation, drying and heat curing. Once the array chip has been prepared in this way, the ligands can be deposited onto the active surface.

At least one scribe line is scribed into the wafer substrate. As used herein, the term "scribe line" refers to a channel that is cut into the wafer substrate. Scribe lines are sometimes referred to as "streets" in the art. A scribe line does not pass all the way through the substrate. Preferably, a scribe line passes approximately 50% of the way through the substrate, more preferably approximately 30%. The purpose of the scribe line is to provide a line of weakness along which the wafer substrate can be broken to form at least one individual array chip. The breaking of a wafer substrate into chips is commonly referred to as "dicing" in the art.

In a preferred embodiment, the scribe lines act not only as lines of weakness along which the wafer can be diced into individual array chips, but also act as reference marks to determine the position of deposited ligand on the wafer. This embodiment is described in more detail below. Preferably, the position of the ligand on the wafer is determined by using the scribe lines as reference marks, before the wafer is diced into array chips. By detecting the position of the ligand in this way, it is possible to detect those areas on the wafer where deposition does not meet the required standard. This provides an early opportunity to remove sub-standard array chips from the production process.

Scribing a substrate using a laser is the preferred method. Laser scribing is well known in the art. One example of a suitable system involves a CO₂ laser which is pulsed to create a number of closely-spaced holes through, or partly through, the substrate. Preferably, the laser is computer-controlled. A general review of (silicon) microchip production using lasers may be found in Masters A, Photonics Spectra 2003; December.

One surface of the wafer substrate is scribed, namely the reverse of the wafer substrate surface that comprises the ligand. For the avoidance of doubt, a planar substrate comprises two main surfaces. One of these surfaces is to be scribed and the other, i.e. the active surface will have the ligand deposited onto it. Scribing and depositing onto opposite sides of the wafer substrate avoids any substrate material, that is sputtered from the scribe line during scribing, from interfering with the ligand. For some substrate materials, such as many ceramics, the scribe lines will not be visible from the side which is not scribed. However, if the substrate is transparent or translucent (and brightly lit), the scribe lines may be visible from both sides of the substrate.

The at least one scribe line may be a single line in the centre of a substrate. Preferably, the scribing comprises a plurality of lines, more preferably the plurality of lines form a grid on the wafer substrate. As used herein, the term "grid" refers to a plurality of lines that cross each other at least once. Preferably, the grid comprises at least one line that is orthogonal to at least one other line. The grid forms a plurality of discrete areas on the substrate, wherein each square of the grid can form one array chip when the wafer substrate is diced along the scribed lines. Preferably, ligand is deposited into at least one of the discrete areas. If the ligand falls outside the discrete area, an error has occurred during the deposition process.

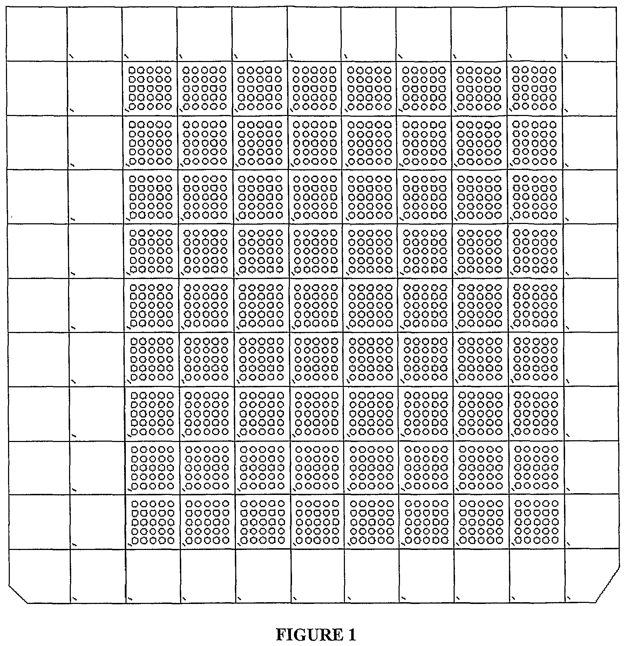

In a preferred embodiment, each discrete area of the grid defines one array chip, and a plurality of ligand depositions are "spotted" into each discrete area that is defined by the grid, for example as illustrated by Figure 1.

As used herein, the term "ligand" refers to any molecule that is deposited onto a substrate, for use in an analyte detection assay; the ligand is capable of binding to an analyte under study. Preferably, the ligand is a biological molecule. The skilled person will understand that the term "biological molecule" refers to nucleic acids, amino acids, lipids and carbohydrates, and any polymer or combination thereof, including oligonucleotides, polynucleotides, peptides and proteins. For the avoidance of doubt, the term "biological molecule" as used herein includes drugs, i.e. molecules that affect biological processes. The arrays manufactured according to the invention may be used to detect the presence of performance enhancing drugs, such as erythopoietin and anabolic steroids, in athletes or to detect the presence of an illegal or illicit substance in a sample. Such drugs will be apparent to one skilled in the art. A drug with a harmful effect, e.g. a poison, is also included within the scope of the invention. Allergens may also be detected. Accordingly, the ligand deposited on the substrate will interact or bind to a drug or allergen under study. Antibodies are the preferred ligand. The skilled person will realise that the ligand cannot be an antibody with affinity for the specific dye being used.

The deposition of ligands onto substrates such as wafer substrates is well known in the art. When creating an array, the deposition process is commonly known as "spotting", and results in the deposition of a plurality of "spots" of ligand on the wafer substrate.

Immobilisation of ligands is well-known in the art and may be carried out by covalent (e.g. a linker molecule) or non covalent means. Methods for immobilising biological molecules onto biochips are particularly well known. Any method of depositing the ligand onto the wafer substrate may be used, including contact and noncontact arraying techniques.

To ensure that the stability and activity of the ligand is not adversely affected by the spotting process, it is preferred that the deposition solution comprises a buffer. Spotting buffers are well known in the art and are usually water-based mixtures comprising bases, acids, various sodium salts, sugars, polysaccharides, surfactants, pH stabilisers, amino acids and proteins. pH is normally adjusted according to requirements. The appropriate buffer to be used will be apparent to one skilled in the art. The morphology of a spot can be controlled by choosing appropriate additives to the spotting buffer and choosing the correct environmental conditions during and after spotting, for example as explained by Deegan et al, *Supra* and Delehanty and Ligler, *Supra.*

In a preferred embodiment, a visible dye that does not interact with the ligand is added to the deposition solution comprising the ligand. This permits simple visualisation of each area of deposition and, because the dye does not interact with the ligand, the activity and specificity of the ligand is not adversely affected.

According to a preferred embodiment of the invention, a visible dye that does not interact chemically with the ligand that is to be deposited is added to the deposition solution. By "does not interact chemically" it is meant that no chemical reaction, resulting in the change in the chemical structure of the ligand or dye, or the production of a product, occurs. The term is intended to exclude any covalent or ionic interaction between the dye and the ligand. Use of a non-reacting dye results in the ligand being unaffected by the presence of the dye. In particular, the ligand is not adversely affected by the dye, i.e. the ligand's physical and chemical characteristics remain unchanged. This is particularly important when the ligand is a biological molecule, where the activity and specificity of the molecule are crucial to the success of any assay. The dye also does not interfere with the ability of the ligand to attach to the substrate.

The dye is visible. This allows simple visual detection, for example using a camera. As used herein the term "visible" refers to a dye that is detectable by a cameras and results in a deposit that can be distinguished from the surrounding substrate. Preferably, the visible dye absorbs light in the visible range, i.e. the portion of the electromagnetic spectrum from approximately 400 to 700 nm wavelength. As will be appreciated by one skilled in the art, the addition of a dye with absorption between approximately 400 nm to 700 nm produces a coloured spotting buffer solution and therefore a coloured deposit ("spot"). The dye may alternatively absorb at a wavelength outside those visible to the human eye but which is detectable by a camera. For example, solid state cameras (e.g. CCD-based) have the potential to detect across a broader range of wavelengths than those visible to the human eye, from near UV at one end of the spectrum to near infrared at the other end of the spectrum. Other detectors and/or cameras are known that can detect these, and other, parts of the electromagnetic (optical) spectrum. Dyes that absorb outside the visible part of the electromagnetic spectrum are therefore also within the scope of the current invention. Suitable dyes are available which absorb in the near infrared region of the spectrum (700 nm-1600 nm). A dye that absorbs in the range of wavelengths normally used for fluorescence (usually in the UV range) can be used. Observation at the illumination rather than the fluorescing wavelength will result in dark spots against a brighter background (the assay substrate). Therefore, the fluorescent characteristic of a dye according to this embodiment is not utilised (i.e. the emission of a wavelength at lower energy than the wavelength absorbed, is not detected).

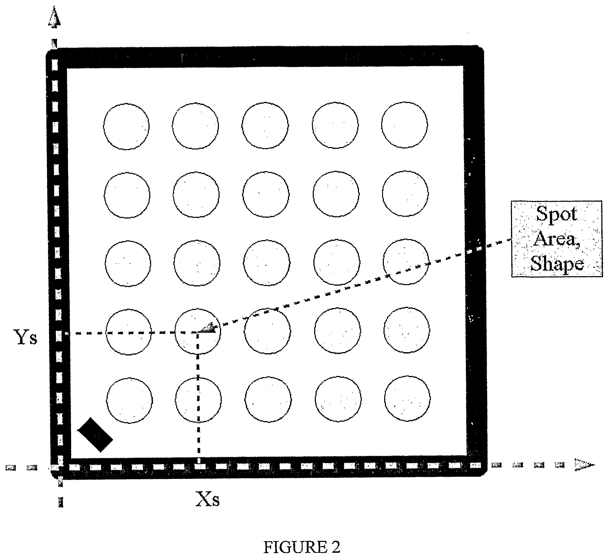

For the avoidance of doubt, the absorption of a particular wavelength is detected according to the current invention, not the emission of a wavelength. For example, if light of a specific wavelength (colour) is absorbed by a dye, the deposited spot containing the dye will appear coloured (a different colour to the absorbed light) or black against the background. If the spots and substrate are illuminated with a Blue (470 nm) LED which is absorbed by the dye but reflected by the substrate, then the spots appear black against a blue background. See, for example, Figure 2. Alternatively, if the spots are illuminated by white light (wide range of wavelengths) then reflected light of certain colour (band of wavelengths) will be observed. The spot will appear coloured, possibly against a white or coloured background depending on the absorbance characteristics (in effect the colour) of the substrates. In white light, if red is absorbed by the spots then the spots will appear green if observed by a colour camera, dim if observed by a black/white camera and black if observed by a camera fitted with a narrow band filter. Therefore, the illumination and detection system could use, for example, (1) a narrow band source and wide bandwidth camera, (2) white light (white bandwidth) source and camera with narrow band filter at the dye absorbing wavelength, (3) a white light or narrow band source and colour camera, or (4) narrow waveband source and narrow waveband band camera.

The physico-chemical properties of the dye are such that no detriment is caused to the specific activity of the ligand in solution or when immobilised onto the surface of the substrate. The dye is preferably added to the ligand prior to deposition onto a substrate. However, the dye may be added to the ligand simultaneously with deposition, or even immediately after deposition (but prior to the deposit drying).

Any dye that does not interact with the ligand may be used. Different ligands will require different dyes to ensure that no chemical interaction occurs, as will be apparent to one skilled in the art. A single dye, or a plurality of dye types may be used.

Preferably, the dye carries a negative charge especially at pH>7, (i.e. in basic conditions). When such a dye is used, it is preferred that the spotting buffer is alkaline, so the dye exhibits a negative overall charge. In a preferred embodiment, the dye is a sulphonated acid dye. These dyes contain sulphonic acid groups, which are usually present as sodium sulphonate salts. These groups give the dye molecules a negative charge and dyes comprising a high proportion sulphonic acid groups are preferred. It is further preferred that the sulphonated acid dye additionally contains phenol or carboxyl groups or other anionic auxochromes. Sulphonated dyes possessing the following physico-chemical properties are preferred embodiments:
1) Dyes that are not reactive;
2) Dyes that are ionic, but not basic, which are acid, possessing anionic auxochromes and therefore negative charges;
3) Dyes that are sulphonated of which those with a significant proportion of SO₃Na are most preferred.
4) Dyes that are hydrophilic, soluble and occurring as salts, preferably sodium salts;
5) Dyes possessing in addition to the sulphonic group as an auxochrome one or more of the following auxochromes (according to Gurr's classification-acid colligators): SO₃⁻, SO₃H, COOH, COONa, OH, ONa;
6) Dyes with high absorptivity (extinction coefficient) and strong chromophore are preferable, for example predominantly azo-dyes (or disazo or polyazo, containing - N=N- bond) or aminotriarylmethane or other type of strong chromophore.
7) Dyes that exhibit fluorescence in addition to the above listed properties are also suitable.

Sulphonated acid dyes carry a negative overall charge in basic conditions. Therefore, it is preferred that the ligand to which the sulphonated acid dye is added also carries a negative charge (in basic conditions), as this will preclude the formation of salts between the ligand and dye (i.e. ionic interaction). Further, the dye and ligand both carrying a negative charge will result in electrostatic repulsion and prevent other chemical interactions. It is therefore preferred that sulphonated acid dyes are added to nucleic acid ligands, as nucleic acids carry a negative charge.

When the ligand is a protein, it is also preferred that sulphonated acid dyes are added to the protein in a spotting buffer with a pH above the isoelectric point of the protein. It is well known in the art that above the isoelectric point, proteins exhibit a negative charge and therefore the dye and protein will repel each other precluding chemical interactions. The isoelectric point of many proteins is well known in the art (for example IgG pl approximately 8, BSA pl approximately 5.5). For a protein or peptide with an unknown isoelectric point, the technique of isoelectric focussing allows the pi to be determined.

Sulphonic acid dyes also offer the opportunity to be added to deposition buffers that are already formulated to be optimal for a particular immobilisation strategy without altering pH, as in aqueous solution their weak acid potential is likely to be balanced by the basic potential of the metal salt (Na) ion, therefore the solutions would be close to neutrality, as discussed in Conn H. J., Biological Stains, A Handbook on the Nature and Uses of the Dyes Employed in the Biological Laboratory, ed. Lillie R. D., Williams and Wilkins, Baltimore, 1977.

Azo dyes, which demonstrate the highest absorptivity (extinction) coefficients, are preferred as they provide a strong colour contrast. However, dyes with other chromophores, for example the indicator dye Xylenol Orange, which has an aminotriarylmethane chromophore, may also be used. Any colour dye may be used, although it is preferred that dyes in the yellow, orange and red part of the spectrum are used. Non-limiting examples of dyes which are suitable for use in the invention are (on the basis of the above criteria and as a result of experimental work, order alphabetical): Acid Red 1 (textile and plastic dye, ink and soap pigment), Amaranth (food colouring), Brilliant Yellow (indicator), Chromotrope R (textile, plastics dye, biological stain), Orange G (biological stain), Sunset Yellow (food dye) and Tartrazine (food and textile dye). Some dyes, such as Orange II or Ponceau S, Fast Yellow, Crocein Orange, Ethyl Orange were found to be non-interfering with the chemiluminescent micro-spot immunoassay but produced a pale colour of spotting buffer salts on the substrate. However, this may be useful in some embodiments and these dyes are also within the scope of the invention. Some of the examples of chemical structures of dyes and the relevant data are shown in Table 1; the data on structure, molecular weight and solubilities are taken from Green F. J., The Sigma-Aldrich Handbook of Stains, Dyes and Indicators, Aldrich Chemical Company Inc., 1990.

Due to differences in chemical structures between individual dyes within a class, there can be some differences in their performance in a micro-spot, particularly where detail of crystallization pattern of the buffer components is concerned. The best overall performance was observed with the dyes positioned closer to the top of Table 1. For example, Brilliant Crocein MOO lower in the table does not give the best performance compared to other dyes as the content of anionic auxochromes is not as high as in Tartrazine or Chromotrope 2R. In addition to surface tension, which plays a major role in definition the micro-spot footprint, a combination of more subtle factors determines the distribution of solutes' (biomolecules, salts etc.) density profile across the spot. Which dye is most suitable for a specific analyte therefore depends on the ligand and conditions, and will be apparent to one skilled in the art.

Once deposited onto the wafer substrate, the visible dye generates contrast between the deposit and the substrate, based on the difference in the light absorption between the dye and the substrate. The specific dye which is used is therefore partially dependent on the optical characteristics of the substrate; it will be appreciated by one skilled in the art that the intensity of colour demonstrated by the dye is important.

The coloured deposits containing the dye are preferably detected against a contrasting background, which allows close-up imaging either in reflection or transmission modes. Optical imaging of the dye-containing deposit can be performed immediately after deposition. The deposit may be partially or fully dried. The deposits can be monitored while the substrate is in the deposition apparatus ("spotting deck") or in any kind of atmospheric chamber that is required to manufacture the array. The deposits may also be detected at any stage after the substrates leaving the spotting apparatus or atmospheric chamber.

Imaging may be carried out using any optical detection or inspection equipment. A typical assembly comprises a camera with a suitable spacial resolution, a uniform and stable visible light source such as an LED array and suitable filters, as will be appreciated by one skilled in the art. Alternatively, gel stain imaging or any other imaging equipment for colorimetric detection, followed by image analysis, may be used. Qualitative or quantitative methods may be used, it is preferred that quantitative information is generated to ensure high quality control. It will be apparent to one skilled in the art that illumination and imaging in the visible range allows for high speed inspection of deposits, in particular on an array, at relatively low cost.

The deposited ligand is monitored to detect that it has been deposited correctly. Correct deposition is determined by a predetermined position, size and shape of the deposit. In the preferred embodiment wherein each deposit is a spot on an array, it is essential that each spot is in the correct position on the substrate, is of the correct size and the correct shape.

**TABLE 1.**

| **Dye** | **Structure** | **F.W.** | **Solubility in water, mg/ml** |
|---|---|---|---|
| **Tartrazine (Acid Yellow 23)** | 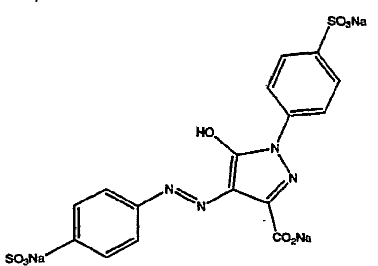 | 534.37 | 300 |
| **Chromotrope 2R (Acid Red 29)** | 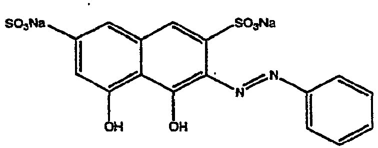 | 468.37 | 200 |
| **Acid Red 1 (Amido Naphthol Red G)** | 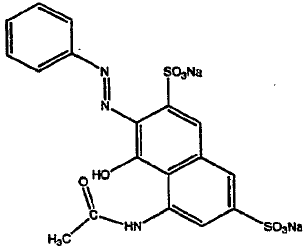 | 509.43 | 60 |
| **Brilliant Yellow (Direct Yellow 4)** | 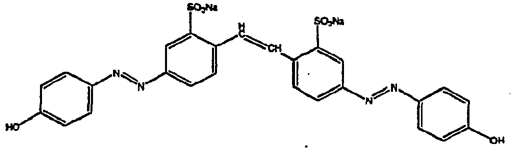 | 624.56 | 1 |
| **Brilliant Crocein MOO (Acid Red 73, Woodstain Sacrlet)** | 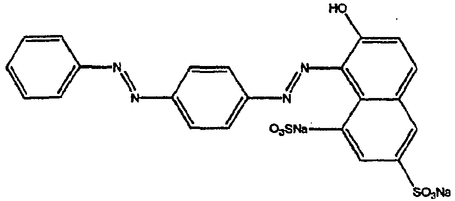 | 556.49 | 50 |
| **Crocein Orange (Acid Orange 12)** | 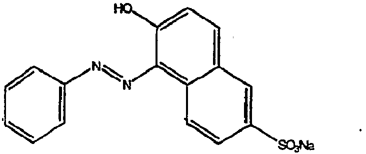 | 350.33 | 40 |

A dye according to the invention does not chemically interact with the ligand and can be removed simply once the deposition of the ligand has been monitored. It is not essential that the dye is removed, although it may be beneficial, so that the dye molecules do not interfere with any subsequent analysis procedure. The dye may be removed from the deposited ligand during a post-deposition wash or upon application of a blocking solution. A separate step specifically to remove the dye is not required. Removal of the dye from the wafer substrate surface does not affect the immobilisation of the ligand to the surface, which is retained (for subsequent analysis).

The at least one scribed line can also function as a pre-defined reference mark on the wafer substrate, to aid the detection of the correct deposition of the ligand onto the wafer substrate. The scribed line defines an area on the wafer substrate. Once the ligand has been deposited onto the substrate, the position of the deposited ligand in relation to the scribed line can be detected. In a preferred embodiment, the scribed lines define a grid on the wafer substrate; each square of the grid is a potential assay chip. If detection of the ligand and scribed line indicates that the ligand is not within the defined area, an error is likely to have occurred in the deposition process and the deposit is not in the correct place. Alternatively, the scribed line can define an area in which the ligand must not be deposited.

The scribed lines may be detected using any suitable detection device. In a preferred embodiment, the substrate is mounted on an XY stage which is moved under control of a computer. A narrow beam (point) laser source and sensor device focusses a laser spot on the scribed side of the substrate. In a preferred embodiment the laser source and detection system is a complete module or sensor; an example of a suitable system is the Keyence LV-H37 small spot transmitter/receiver module, which provides a small spot diameter of ∼50um at a detecting distance of 70+/-15mm.

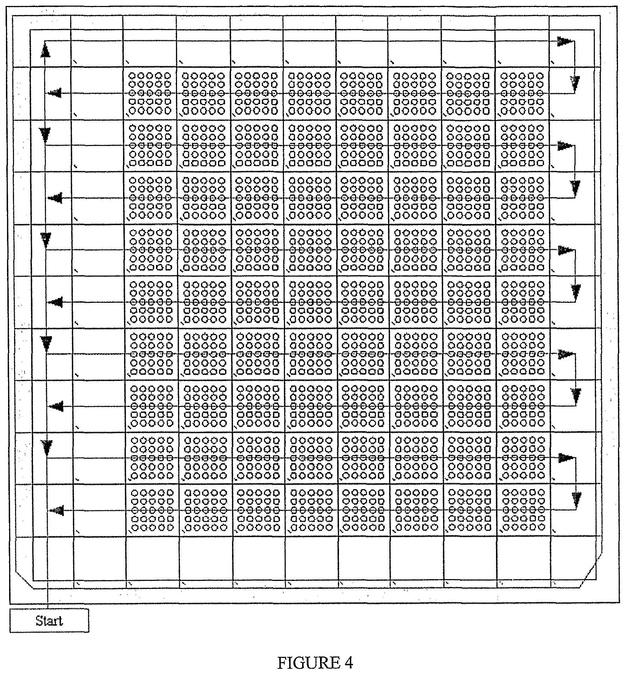

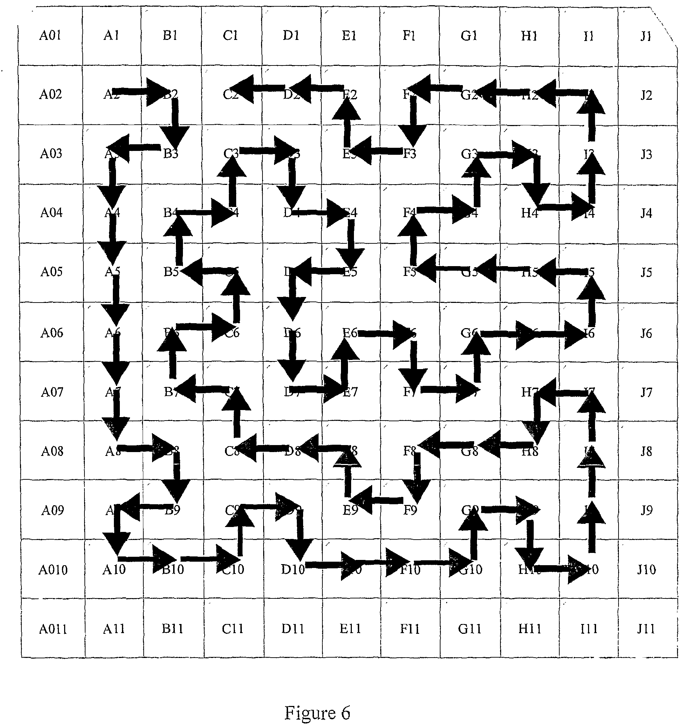

In a preferred embodiment, the position and direction of the laser and sensor are fixed and the substrate, on the stage, is moved with respect to the fixed laser and sensor. As the substrate is moved in X and Y directions, a change in the reflected radiation is observed as the laser spot moves across a scribed line, thereby determining the scribe line intersection position with respect to the stage position at that instant. Figures 4 and 6 illustrate potential patterns of movement of the substrate with respect to a fixed laser and sensor. In Figure 4, the zigzag pattern across the chip provides 10 intersections of the vertical scribe lines but only two intersections of the horizontal scribe lines, while the pattern in Figure 6 crosses each scribe line at least four times during one complete scan of each biochip area on the substrate. It will be apparent to one skilled in the art that any pattern of detection that crosses the scribe lines will allow the position of the scribe lines to be determined.

Detection of the scribed line preferably occurs simultaneously with detection of the deposition of the ligand. However, it is within the scope of the invention that the reference mark and deposited ligand are monitored separately and subsequent analysis is used to compile the information, for example overlaying two separate images.

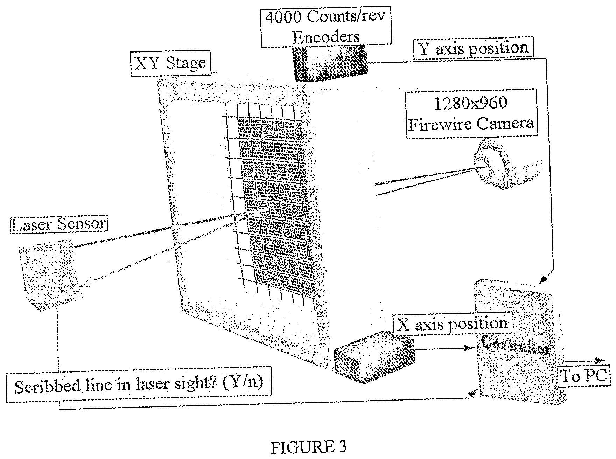

A preferred method involves the simultaneous detection of the at least one scribed line on one side (i.e. one surface) of the wafer substrate and detection of the deposited ligand on the opposite surface of the wafer substrate, as depicted in Figure 3. This detection, before the wafer is diced into array chips, allows defects in ligand deposition to be detected early in the chip production process. In Figure 3, there are three coordinate or reference sub-systems which must be aligned: the XY stage with its positioning system, the camera with its own coordinate system, and the laser sensor. Once aligned, the camera and laser sensor are locked in position and the XY stage moves in relation to these. Alignment of the laser sensor (or other sensor for detecting the reference marks) and the camera (or other device for detecting the deposited ligand) is preferred so that the camera and laser sensor focus on the same region of the substrate. A suitable alignment process involves firstly aligning the camera and the XY stage using a test (distortion) target. Pattern matching enables the camera to be physically rotated until the stage motion in X and Y is parallel to cameras X and Y coordinate system. Alternatively, image processing techniques may be employed to "rotate" the camera image until aligned with the stage. The test (distortion) target also enables the perpendicularity of the XY stage relative to the camera axis to be adjusted until keystone effects are corrected. The second step involves placing a, preferably translucent, substrate on the stage, turning on the laser and detecting the position of the laser spot within the camera coordinate system, for example by image processing. This will define the centre of the detection points.

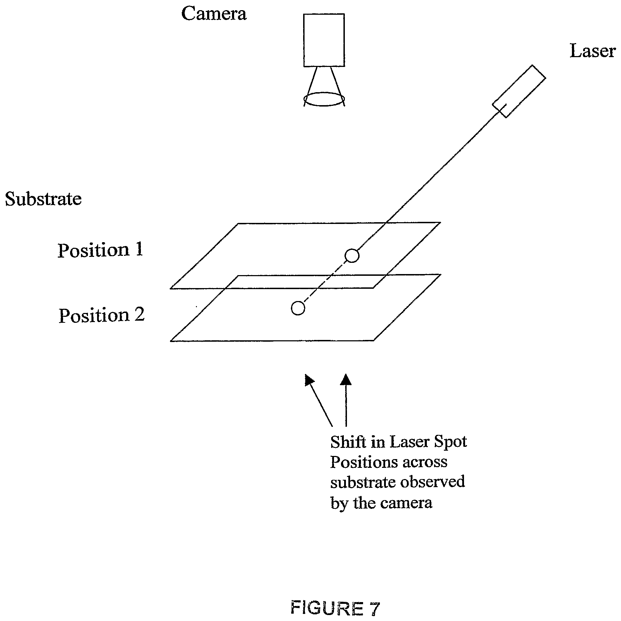

An additional one or more lasers may be used in the set up procedure to determine the separation of the camera from the substrate and adjust the distance between them. In Figure 7, the second laser illuminates one surface (upper or lower) of the substrate with a laser dot at an acute angle to the camera axis. Movement of the substrate towards or away from the camera is therefore observed by a shift in the spot position observed. Observation of the spot image allows adjustments to be made to the distance between the substrate and camera. This will ensure consistency in the dimensional measurements of points/positions across the substrate surface, made by the camera system.

Once set up, the stage and substrate are moved in a scan pattern, for example as illustrated in Figure 6. Stage positions are stored at points of intersection of the laser beam and scribe lines. During the scan, images are taken of the spotted material on the opposite surface to the scribe lines, in each biochip area. These operations enable the scribe lines, subsequently each chip corner position and ultimately the positions of each spot within each discrete area (i.e. chip) of the substrate to be determined by computation at the end of a complete scan of the substrate or part of the substrate. This system has the advantage of having the ability to detect deposited ligand and reference marks using a single camera (the reference marks being detected by laser). The lack of a second camera simplifies the system by simplifying the combination of reference marks and ligand position data. The laser detector operates in parallel with the (single) camera, providing the required accuracy at a lower cost than a second camera.

The invention is further described in the following non-limiting Example.

### Example

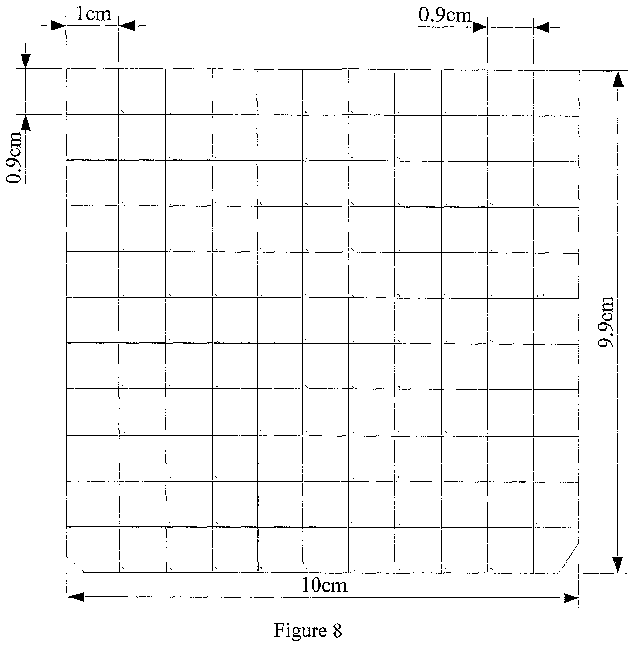

The raw substrate (alumina) was laser scribed according to the pattern presented in Figure 8. The generated pattern lines are going to be used as breaking points in the dicing process at the end of the manufacturing process.

The laser scribing process drills blind, conical holes, approximately one third through the substrate.

All regions at the periphery of the ceramic plate are being used during the manufacturing process for handling, leaving a useful central area (the shaded part in Figure 8) of 81 individual 9x9 mm square regions.

During the laser scribing process a small mark was placed on each individual region to allow for visual identification of the active side of the substrate.

A cleaning process was used to remove any debris, followed by a silanation process, drying and heat curing.

Antibody (ligand) concentrations in the spotting solutions tested in the development of this manufacturing technique ranged from 0.033 to 1.2 mg/ml. The spots created on the surface were typically about 300 µm in diameter. Dye concentrations in the spotting solution ranged from 0.5 to 10 mg/ml. In most cases coloured spots were highlighted well enough for automated imaging and image analysis at 1 mg/ml of dye in spotting buffer. The maximum amount of dye that is acceptable for the solution depends on how much of other salts is already incorporated and how the solubility of the dye is affected by the other components present in the buffer.

The calculated ratios of dye molecules per antibody molecule in the typical buffers ranged from a minimal number of approximately 260 to exceeding 8x10⁴ in some cases. When taking molecular packing considerations into account these numbers indicate that if dye molecules demonstrated attraction to the antibodies they would be expected to surround them quite densely and the consequent detriment to activity and specificity would be expected. So in this sense the quantity of dyes added cannot be seen as 'negligible'. Also when the hypothetical density of packing of dye molecules within a typical spot area is estimated, this density turns out to be much higher by a factor of at least 10⁹ than that corresponding to a theoretical single layer, which means that if the dye itself was actually binding to the surface or in some other way interfering with the biomolecule binding to the surface, this would manifest itself in a dramatic detriment to the assay signal.

The spotting solution was spotted using an off the shelf microarrayer, creating up to 81 individual arrays on top of each square region on the substrate. As some spotting equipment uses 8 tips spaced at 9mm distance, in some cases is more cost-effective to generate a total of 72 (8x9) arrays instead of 81 arrays per substrate.

After spotting, a primary drying process was employed, followed by an imaging stage.

At the imaging stage, the substrate was loaded onto the imaging deck (Figure 3), with the active (spotted) side facing the camera and the scribed side facing the scribing detection laser, then was transported according to the pattern in figure 6.

Every time one array is located inside the field of view of the camera, the scribing detection laser was turned off, and a camera acquisition was triggered.

An image-processing algorithm was employed to automatically analyse this captured image and determine the size, shape and location of each spot, in reference to the origin that is being set at the calibration stage as being the centre of the scribing detection laser spot.

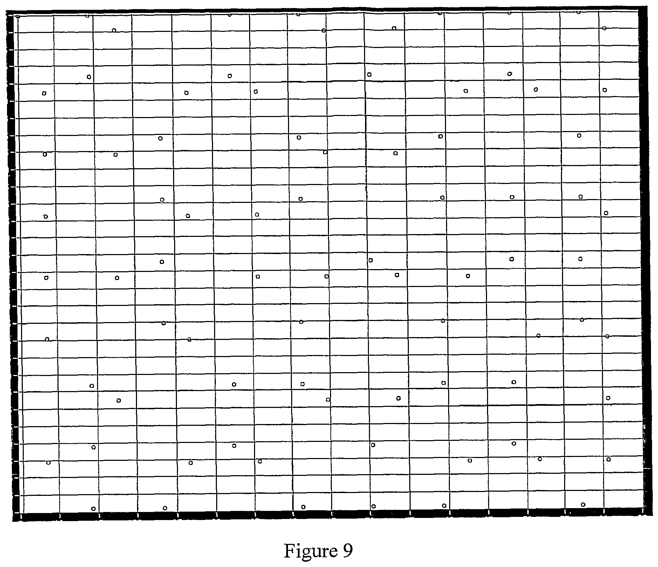

Subsequently the scribing detection laser was turned on and the substrate moved to the next region. Every time a scribed line crosses the laser sensor, it triggers a position capture on the motion controller. This process was repeated for the whole substrate. All the positions detected as crossing points are stored for each sheet, as shown in figure 9.

Once the whole sheet was scanned and all data obtained, a line-fitting algorithm was employed to fit each vertical / horizontal scribed line to the corresponding crossing point. The intersection of these lines corresponds to the individual array chip corners. For example, consider the following intersection points A2-B2, B3-A3, A8-B8, B9-A9, A10-B10. By fitting a line through these points the position of the line dividing column A and column B can be determined.

In the same way the position of the laser sensor must be determined with reference to the substrate. This is being done in a similar manner, by fitting and intersecting corresponding lines. For example, to determine the position of the laser sensor at the imaging moment with respect to chip A10, we would fit A3-A4, A4-A5, A6-A7, A7-A8, A9-A10 to generate the vertical component, followed by A10-B10, B10-C10, D10-E10, E10-F10, G10-G10, H10-I10 to generate the horizontal component. By intersecting those two lines we can determine the position of the laser sensor; that corresponds to the origin for the camera coordinate system for the array A10.

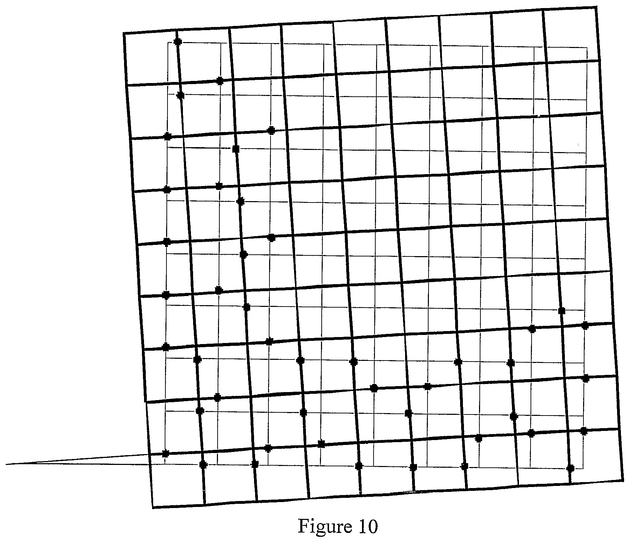

Further more, the angle by which the ceramic substrate was offset with reference to the scanning pattern was determined in a similar way, by fitting the appropriate points, according to figure 10.

In practice the offset is quite close to zero, therefore the offset angle is being determined for perpendicular lines was calculated as the deviation from 90°. For example A3-A4, A4-A5, A6-A7, A7-A8, A9-A10 can be fitted to generate the vertical component, followed by A9-A10, C10-C9, D9-D10, G10-G9, H9-H10, 110-19 to generate the horizontal component. The angle between the two fitted lines is calculated, and the deviation from 90° represents the amount by which the plate is rotated with respect to the motion pattern. However the motion pattern axis are parallel to the camera axis (by calibration) therefore the offset angle represents the amount by which each array is being rotated with reference to the camera.

At this point for every array the measurement reference system was rotated by the plate offset angle and translated from the scribing detection laser to the array corner. In this way positioning measurements become absolute with reference to the array geometry and spotting quality is being assessed against the array spotting specification, and any non-conforming array was marked for rejection in the downstream process, at the stage where the substrate is going to be broken into individual arrays.

## Claims

1. A method of manufacturing an array chip, comprising the steps of:
i) depositing at least one ligand onto the active surface of a wafer substrate having at least one scribe line, the active surface being the reverse of the surface comprising the at least one scribe line;
ii) detecting the position of the deposited ligand in relation to the at least one scribe line;
iii) determining whether the ligand has been deposited correctly in relation to the at least one scribe line; and
iv) breaking the wafer substrate along the at least one scribe line, to produce at least one array chip.

2. A method according to claim 1, wherein the array chip and wafer substrate are ceramic.

3. A method according to claim 1 or claim 2, wherein the scribe line has been produced by a laser.

4. A method according to any preceding claim, wherein in step (i) a deposition solution comprising the ligand and a visible dye that does not interact chemically with the ligand is deposited onto the wafer.

5. A method according to claim 4, wherein step (ii) is carried out by detecting the position of the deposited ligand and visible dye solution.

6. A method according to claim 4 or claim 5, wherein the visible dye is negatively charged.

7. A method according to any preceding claim, wherein the at least one scribe line forms a grid.

8. A method according to any of claims 4 to 7, wherein correct deposition of the solution comprising the ligand and visible dye requires the correct, predetermined position, size and shape of the deposit.

9. A method according to claim 8, wherein the predetermined position of the solution is defined by the at least one scribe line.

10. A method according to any preceding claim, wherein the dye does not interact covalently or ionically with the ligand or the substrate.

11. A method according to any preceding claim, wherein the ligand is a biological molecule.

12. A method according to claim 11, wherein the ligand is a protein or a peptide.

13. A method according to claim 12, wherein the protein or peptide is in a buffer with a pH greater than the isoelectric point of the protein or peptide.

14. A method according to claim 11, wherein the ligand is a polynucleotide.

15. A method according to claim 11, wherein the ligand is an organic molecule.

16. A method according to claim 15, wherein the ligand is a steroid.

17. A method according to any preceding claim, wherein the dye is a sulphonated acid dye.

18. A method according to any preceding claim, wherein the dye is hydrophilic.

19. A method according to any preceding claim, wherein the dye is fluorescent.

20. A method according to any preceding claim, wherein after monitoring the ligand on the substrate, the dye is removed.

21. A method according to claim 20, wherein the dye is removed by contact with an aqueous solution.

## Patentansprüche

1. Verfahren zur Herstellung eines Array-Chips, folgende Schritte umfassend:
i) Ablagern mindestens eines Liganden auf die aktive Oberfläche eines Wafersubstrats mit mindestens einer Ritzlinie, wobei die aktive Oberfläche die Rückseite der Oberfläche ist, welche die mindestens eine Ritzlinie;
ii) Erkennen der Position des abgelagerten Liganden in Bezug auf den mindestens einen Ritzrahmen;
iii) Ermitteln, ob der Ligand in Bezug auf die mindestens eine Ritzlinie korrekt abgelagert worden ist; und
iv) Brechen des Wafersubstrats entlang der mindestens einen Ritzlinie, um mindestens einen Array-Chip zu produzieren.

2. Verfahren nach Anspruch 1, wobei der Array-Chip und das Wafersubstrat keramisch sind.

3. Verfahren nach Anspruch 1 oder Anspruch 2, wobei die Ritzlinie von einem Laser erzeugt worden ist.

4. Verfahren nach einem vorhergehenden Anspruch, wobei in Schritt (i) eine Ablagerungslösung, die den Liganden und einen sichtbaren mit dem Liganden nicht chemisch reagierenden Farbstoff umfasst, auf dem Wafer abgelagert wird.

5. Verfahren nach Anspruch 4, wobei der Schritt (ii) durch Erkennen der Position des abgelagerten Liganden und der sichtbaren Farbstofflösung ausgeführt wird.

6. Verfahren nach Anspruch 4 oder Anspruch 5, wobei der sichtbare Farbstoff negativ geladen ist.

7. Verfahren nach einem vorhergehenden Anspruch, wobei mindestens eine Ritzlinie ein Gitter bildet.

8. Verfahren nach einem der Ansprüche 4 bis 7, wobei die korrekte Ablagerung der Lösung, welche den Liganden und den Farbstoff umfasst, die korrekte, vorbestimmte Position, Größe und Form der Ablagerung erfordert.

9. Verfahren nach Anspruch 8, wobei die vorbestimmte Position der Lösung durch die mindestens eine Ritzlinie definiert ist.

10. Verfahren nach einem vorhergehenden Anspruch, wobei der Farbstoff nicht kovalent oder ionisch mit dem Liganden oder dem Substrat reagiert.

11. Verfahren nach einem vorhergehenden Anspruch, wobei der Ligand ein biologisches Molekül ist.

12. Verfahren nach Anspruch 11, wobei der Ligand ein Protein oder ein Peptid ist.

13. Verfahren nach Anspruch 12, wobei sich das Protein oder Peptid in einem Puffer mit einem pH größer als der isoelektrische Punkt des Proteins oder Peptids befindet.

14. Verfahren nach Anspruch 11, wobei der Ligand ein Polynucleotid ist.

15. Verfahren nach Anspruch 11, wobei der Ligand ein organisches Molekül ist.

16. Verfahren nach Anspruch 15, wobei der Ligand ein Steroid ist.

17. Verfahren nach einem vorhergehenden Anspruch, wobei der Farbstoff ein sulfonierter Säurefarbstoff ist.

18. Verfahren nach einem vorhergehenden Anspruch, wobei der Farbstoff hydrophil ist.

19. Verfahren nach einem vorhergehenden Anspruch, wobei der Farbstoff fluoreszierend ist.

20. Verfahren nach einem vorhergehenden Anspruch, wobei der Farbstoff, nach Überwachung des Liganden auf dem Substrat, entfernt wird.

21. Verfahren nach Anspruch 20, wobei der Farbstoff durch Kontakt mit einer wässrigen Lösung entfernt wird.

## Revendications

1. Procédé de fabrication d'une puce à réseau, comportant les étapes consistant à :
i) déposer au moins un ligand sur la surface active d'un substrat de tranche ayant au moins un chemin de découpe, la surface active étant l'inverse de la surface comportant ledit au moins un chemin de découpe ;
ii) détecter la position du ligand déposé par rapport audit au moins un chemin de découpe ;
iii) déterminer si le ligand a été déposé correctement par rapport audit au moins un chemin de découpe ; et
iv) casser le substrat de tranche le long dudit au moins un chemin de découpe, pour produire au moins une puce de réseau.

2. Procédé selon la revendication 1, dans lequel la puce de réseau et le substrat de tranche sont de la céramique.

3. Procédé selon la revendication 1 ou la revendication 2, dans lequel le chemin de découpe a été produit par un laser.

4. Procédé selon l'une quelconque des revendications précédentes, dans lequel, au cours de l'étape (i), une solution de dépôt comportant le ligand et un colorant visible qui ne permet aucune interaction chimique avec le ligand est déposée sur la tranche.

5. Procédé selon la revendication 4, dans lequel l'étape (ii) est effectuée en détectant la position de la solution de ligand et de colorant visible ayant été déposée.

6. Procédé selon la revendication 4 ou la revendication 5, dans lequel le colorant visible est chargé négativement.

7. Procédé selon l'une quelconque des revendications précédentes, dans lequel ledit au moins un chemin de découpe forme une grille.

8. Procédé selon l'une quelconque des revendications 4 à 7, dans lequel le dépôt correct de la solution comportant le ligand et le colorant visible nécessite les position prédéterminée, taille et forme correctes du dépôt.

9. Procédé selon la revendication 8, dans lequel la position prédéterminée de la solution est définie par ledit au moins un chemin de découpe.

10. Procédé selon l'une quelconque des revendications précédentes, dans lequel le colorant ne permet pas une interaction covalente ou ionique avec le ligand ou le substrat.

11. Procédé selon l'une quelconque des revendications précédentes, dans lequel le ligand est une molécule biologique.

12. Procédé selon la revendication 11, dans lequel le ligand est une protéine ou un peptide.

13. Procédé selon la revendication 12, dans lequel la protéine, ou le peptide, est dans un tampon ayant un pH supérieur au point isoélectrique de la protéine ou du peptide.

14. Procédé selon la revendication 11, dans lequel le ligand est un polynucléotide.

15. Procédé selon la revendication 11, dans lequel le ligand est une molécule organique.

16. Procédé selon la revendication 15, dans lequel le ligand est un stéroïde.

17. Procédé selon l'une quelconque des revendications précédentes, dans lequel le colorant est un colorant acide sulfoné.

18. Procédé selon l'une quelconque des revendications précédentes, dans lequel le colorant est hydrophile.

19. Procédé selon l'une quelconque des revendications précédentes, dans lequel le colorant est fluorescent.

20. Procédé selon l'une quelconque des revendications précédentes, dans lequel, après une surveillance du ligand sur le substrat, le colorant est retiré.

21. Procédé selon la revendication 20, dans lequel le colorant est retiré par contact avec une solution aqueuse.
